# EUROPEAN PATENT APPLICATION

(11) **EP 2 413 252 A1**
(43) Date of publication of application: **01.02.2012**
(21) Application number: 09842195.1
(22) Date of filing: 23.03.2009
(51) Int. Cl.: G06F 17/30

(54) **METHOD OF RECOMMENDING CONTENT, METHOD OF CREATING RECOMMENDATION INFORMATION, CONTENT RECOMMENDATION PROGRAM, CONTENT RECOMMENDATION SERVER, AND CONTENT-PROVIDING SYSTEM**

(71) Applicant: Fujitsu Limited, Kawasaki-shi, Kanagawa 211-8588 (JP)
(72) Inventor: NAGANO, Reiko, Tokyo 135-8300 (JP); TAMAKI, Kenichi, Kawasaki-shi Kanagawa 211-8588 (JP)
(74) Representative: Ward, James Norman
(86) International application number: PCT/JP2009/055684
(87) International publication number: WO 2010/109581

(57) **Abstract**

Information that a user needs is provided to the user without increasing burden on the user.

A search input item acquisition means (1a) acquires a search input item. A recommended content information creation means (1b) acquires recommendation information records, compares the search input item acquired in a search input item acquisition step with the search input items of the recommendation information records to extract recommendation information having the matched search input item according to their points, and creates recommended content information that links to content indicated by the extracted recommendation information records. A recommended content information providing unit (1c) provides the user with the recommended content information created by the recommended content information creation means (1b).

## Description

### Technical Field

The embodiment discussed herein is related to a content recommending method, a recommendation information creating method, a content recommendation program, a content recommendation server, and a content providing system, for recommending content to a user.

### Background Art

In recent years, thanks to the development and spread of information technology, content is freely available to the general public via the use of information processing apparatuses, such as personal computers, and information technology for network communications, such as the Internet. Examples of content include problem-solving frequently asked questions (FAQs) on a website as after-sales service for products, and help files and troubleshooting tips available online and offline.

In such content that provides information, a user is allowed to carry out a search with keywords within the content, so that user-needed information is provided to the user.

How to provide information, specifically, an order of displaying search results, is important for such content. What is desired is to provide information in a user-friendly manner where, for example, content that a user needs or links to content helpful to the user appears at the top of the search results. To this end, only user-needed information needs to be extracted with great accuracy.

There have been several attempts to extract user-needed information with greater accuracy. For example, the last update date and time of content is focused on to limit a search to only newer information. Another example is that search results are categorized or optimized to user needs.

Patent Document 1 below proposes a method of providing user-needed information to a user.
Patent Document 1: Japanese Laid-open Patent Publication No. 2004-86549

### Disclosure of Invention

### Problem to be Solved by the Invention

According to the technique described in Patent Document 1, a user views a FAQ tip as a result of keyword search, and only if he/she answers "this is not helpful" to a questionnaire for this FAQ tip, other tips (sample questions) appear for displaying "desired content". Therefore, what the user needs to do in order to access needed information (desired content) after start of a keyword search is to carry out the keyword search, check sample questions, and select an appropriate sample question. Therefore, this technique is too complicated for the user.

Furthermore, in this technique, users are supposed to answer questionnaire after each keyword search, and "desired content" for a user is determined on the basis of their answers to the questionnaire. This means that the technique needs users to make evaluations through the questionnaire. This is a time-consuming procedure, so the users may skip the questionnaire. In addition, the accuracy of evaluations is in doubt for the following reasons: not many users answer questionnaire; respondent bias occurs; and evaluations made as answers to the questionnaire may not be reliable due to intentional manipulation and psychological tendency.

In view of the foregoing, it is an object of the present invention to provide a content recommending method, a recommendation information creating method, a content recommendation program, a content recommendation server, and a content providing system, for providing user-needed information without increasing burden on a user.

### Means for Solving the Problems

A disclosed content recommending method causes a computer to perform the following steps: a search input item acquisition step of acquiring a search input item entered by a user to search content available to the user; a recommended content information creation step of acquiring, from a recommendation information storage means, recommendation information records each associating a combination of content viewed and a search input item entered in a session where the content was searched for and viewed with a point given based on the content and the search input item, comparing the search input item acquired in the search input item acquisition step with the search input items of the recommendation information records acquired from the recommendation information storage means to extract recommendation information records having the matched search input item according to points thereof, and creating recommended content information that links to the content indicated by the extracted recommendation information records; and a recommend content information providing step of providing the created recommended content information to the user.

According to the disclosed content recommending method, in the search input item acquisition step, a search input item is acquired. In the recommended content information creation step, recommendation information records are acquired, a comparison is made between the search input item acquired in the search input item acquisition step and the search input items of the acquired recommendation information records, recommendation information records having the matched search input item are extracted according to their points, and recommended content information that links to the content indicated by the extracted recommendation information records is created. In the recommend content information providing step, the recommended content information created in the recommended content information creation step is provided to a user.

### Advantage of the Invention

According to the disclosed content recommending method, recommendation information creating method, content recommendation program, content recommendation server, and content providing system, it becomes possible to provide a user entering a search input item with content having user-needed information on the basis of the same input item entered by other users.

The above and other objects, features and advantages of the present invention will become apparent from the following description when taken in conjunction with the accompanying drawings which illustrate preferred embodiments of the present invention by way of example.

### Brief Description of Drawings

FIG. 1 gives an overview of the embodiment;
FIG. 2 illustrates a system configuration according to the embodiment;
FIG. 3 illustrates a hardware configuration of a web server;
FIG. 4 is a block diagram illustrating a configuration of a content providing system;
FIG. 5 illustrates a configuration of a recommendation table;
FIG. 6 is illustrates a configuration of an access log table;
FIG. 7 is a flowchart illustrating a content recommendation process;
FIG. 8 is a flowchart illustrating the content recommendation process;
FIG. 9 is a flowchart illustrating a recommendation information creation process;
FIG. 10 is a flowchart illustrating the recommendation information creation process;
FIG. 11 illustrates how to give search points and viewing points in each session;
FIG. 12 illustrates how to give order points in each session;
FIG. 13 illustrates order points of each set;
FIG. 14 illustrates destination points of each set;
FIG. 15 illustrates how to create recommendation information records;
FIG. 16 illustrates a search page; and
FIG. 17 illustrates a search result display page.

### Best Mode for Carrying out the Invention

The preferred embodiment of the present invention will be described below with reference to the accompanying drawings.

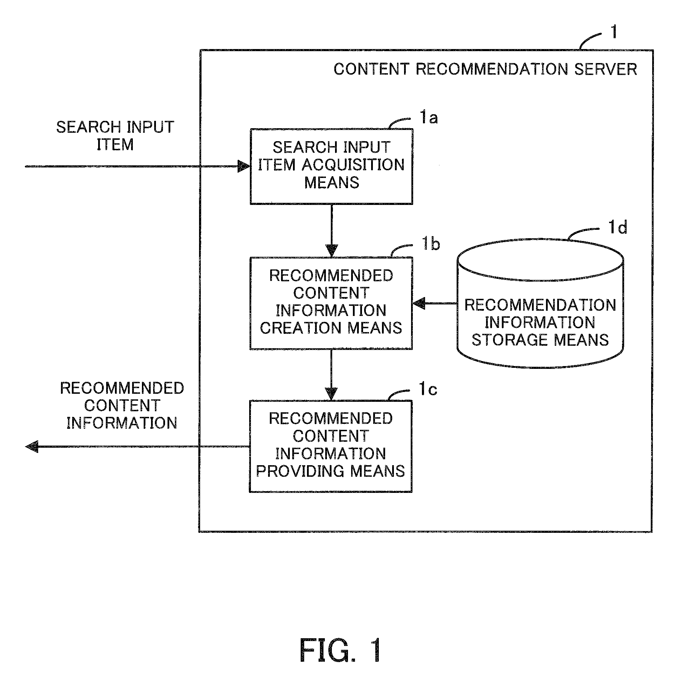

FIG. 1 gives an overview of the embodiment. A content recommendation server 1 illustrated in FIG. 1 provides a user with recommended content information that links to recommended content, on the basis of a search input item entered by the user. The content recommendation server 1 includes a search input item acquisition means 1a; a recommended content information creation means 1b; a recommended content information providing means 1c; and a recommendation information storage means 1d. The content recommendation server 1 is achieved by a content recommendation program which causes a computer to function as the search input item acquisition means 1a, the recommended content information creation means 1b and the recommended content information providing means 1c, and executes a content recommending method.

The search input item acquisition means 1a acquires a search input item entered by a user to search content available to the user. For example, the search input item may be a character string entered by a user for search, such as words like keywords or a sentence. In addition, the search input item may include one or more continuous digits or letters, or a combination thereof, such as a product code. The search input item may be or include information of any type, such as a two-dimensional code, whatever a user can enter.

The content to be provided may be web pages, or other type of content that is usable by users, such as text files, movie files, image files, audio files, analog data, and printed materials.

The recommended content information creation means 1b acquires recommendation information records stored in the recommendation information storage means 1d, compares the search input item acquired by the search input item acquisition means 1a with the search input items of the recommendation information records acquired from the recommendation information storage means 1d, and extracts recommendation information records having the matched search input item according to their points. Next, the recommended content information creation means 1b creates recommended content information that links to content indicated by the extracted recommendation information records.

The recommended content information providing means 1c provides the user with the recommended content information created by the recommended content information creation unit 1b. The recommended content information may be transmitted to the user via a communication line, for example, as data for displaying a web page having URL (uniform resource locator) links. Alternatively, the recommended content information may be provided in a variety of forms that allow users to access recommended content, for example, as an e-mail which has links to the content.

The recommendation information storage means 1d stores recommendation information records each associating a combination (set) of content viewed in a session for content searching and viewing and a search input item used in the session, with points given based on the search input item and the content.

In the content recommendation server 1, the search input item acquisition means 1a acquires a search input item. The recommended content information creation means 1b acquires recommendation information records, compares the search input item acquired by the search input item acquisition means 1a with the search input items of the acquired recommendation information records, extracts recommendation information records having the matched search input item according to their points, and creates recommended content information that links to the content indicated by the extracted recommendation information records. The recommended content information providing means 1c provides the recommended content information created by the recommended content information creation means 1b to the user.

The above content recommendation server 1 makes it possible to provide content having user-needed information to a user entering a search input item, on the basis of the same search input item entered by other users.

The embodiment will be described in detail with reference to the accompanying drawings.

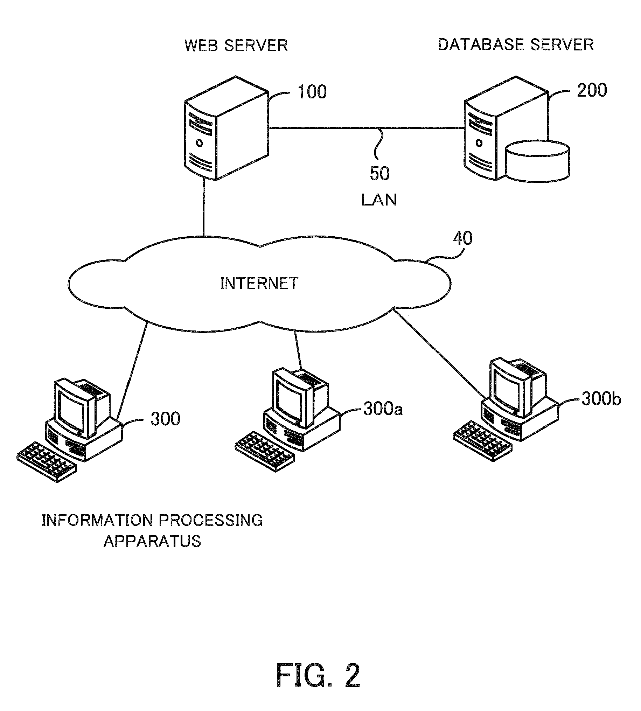

FIG. 2 illustrates a system configuration according to the embodiment. Referring to FIG. 2, there is provided a content providing system in which a web server 100 provides information by transmitting data stored in a database server 200 (for example, data for displaying content to provide information such as FAQs) via the Internet 40 to users' information processing apparatuses 300, 300a, and 300b.

In the content providing system according to the embodiment, the web server 100 which provides information service content is connected via the Internet 40 to the users' information processing apparatuses 300, 300a, and 300b which receive the provided information service content. The web server 100 is also connected via a local area network (LAN) 50 to the database server 200 storing data for the information service content.

Although the web server 100 and the database server 200 are connected via the LAN 50, they may be connected via another communication line such as the Internet or VPN (virtual private network).

The web server 100 provides information service content to a user in such a manner that the web server 100 receives a request for providing content data, from a user's information processing apparatus 300, 300a, 300b (for example, information processing apparatus 300) which functions as a browser, retrieves the requested content data from the database server 200, and then transmits the retrieved data to the user's information processing apparatus 300.

The web server 100 allows a user to search its own information service content available to the user. In addition, the web server 100 predicts and recommends content which is likely to be useful to the user, on the basis of access logs acquired when other users accessed the content and a search input item entered by the user for search, such as a character string.

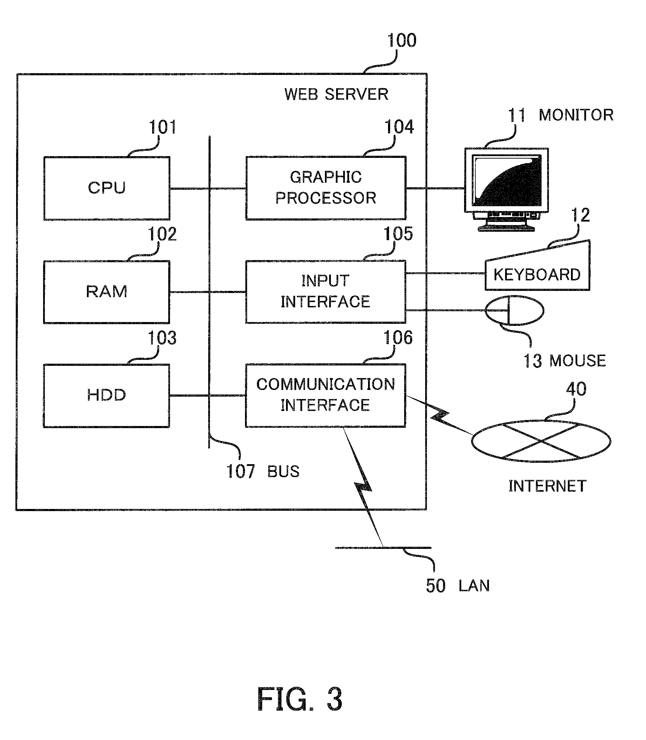

FIG. 3 illustrates a hardware configuration of a web server. A web server 100 illustrated in FIG. 3 is entirely controlled by a CPU (Central Processing Unit) 101. A RAM (Random Access Memory) 102, an HDD (hard disk drive) 103, a graphic processor 104, an input interface 105, and a communication interface 106 are connected to the CPU 101 via a bus 107.

The RAM 102 temporarily stores at least part of the OS (operating system) and application programs to be executed by the CPU 101. The RAM 102 also stores various kinds of data needed for the processing by the CPU 101. The HDD 103 stores the OS and application programs.

A monitor 11 is connected to the graphic processor 104. The graphic processor 104 displays images on the screen of the monitor 11 according to instructions from the CPU 101. A keyboard 12 and a mouse 13 are connected to the input interface 105. The input interface 105 transmits a signal received from the keyboard 12 or the mouse 13 to the CPU 101 via the bus 107.

The communication interface 106 is connected to a network such as the Internet 40 or the LAN 50. The communication interface 106 sends and receives data via the network to and from another computer.

The database server 200, and the information processing apparatuses 300, 300a, and 300b are achieved by the same hardware configuration as that of the web server 100.
This hardware configuration achieves the processing functions according to the embodiment.

Next, a description is given for a configuration of a content providing system.

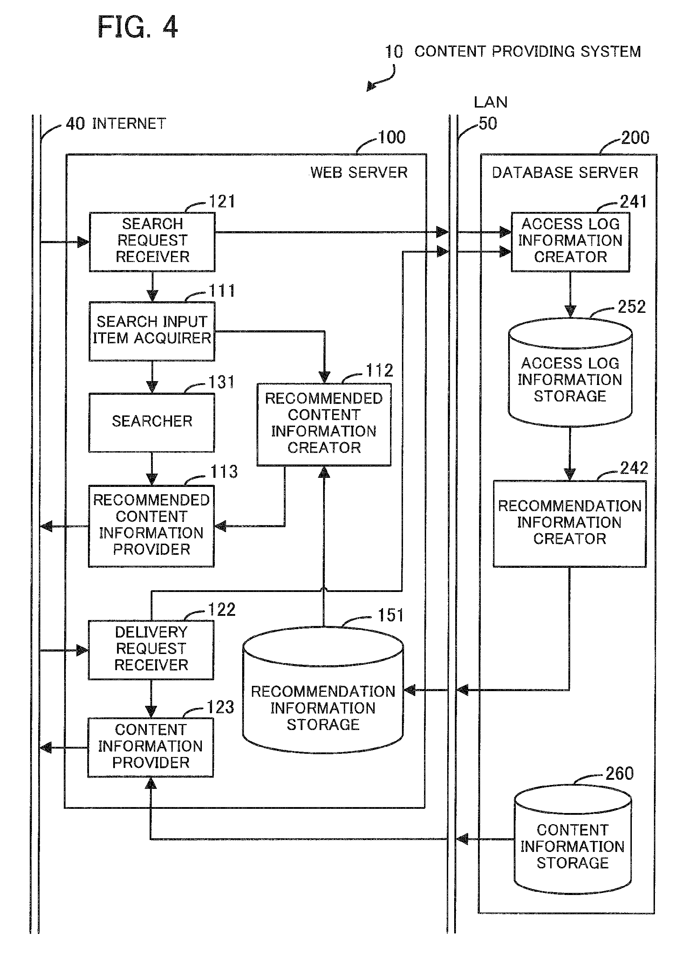

FIG. 4 is a block diagram illustrating a configuration of a content providing system.
A content providing system 10 according to the embodiment is a system that provides web page content having user-needed information in response to a user's request via a communication line. The content providing system 10 includes a web server 100 and a database server 200.

The web server 100 is a computer that recommends content which is likely to be useful to a user in response to a user request for searching content available on the content providing system 10. The web server 100 includes a search input item acquirer 111, a recommended content information creator 112, a recommended content information provider 113, a search request receiver 121, a delivery request receiver 122, a content information provider 123, a searcher 131, and a recommendation information storage 151. These functions of the web server 100 are achieved by causing a computer to execute a content recommendation program.

The web server 100 have connections to the Internet 40 and the LAN 50. The web server 100 is capable of connecting to each of the information processing apparatuses 300, 300a, and 300b (for example, information processing apparatus 300) described with reference to FIG. 2 via the Internet 40. The web server 100 is connected to the database server 200 via the LAN 50.

The search input item acquirer 111 acquires a search input item entered by a user for searching content available to the user, from a search request received by the search request receiver 121. For example, the search input item may be a character string entered by the user for search, such as words like keywords or a sentence. Further, the search input item may include one or more continuous digits or letters, or a combination thereof, such as a product code. Furthermore, the search input item may be or include information of any type, such as a two-dimensional code, whatever a user can enter.

The content provided by the content providing system 10 is assumed to be web pages. However, content of other types usable by users is also applicable, such as text files, movie files, image files, audio files, analog data, and printed materials.

The recommended content information creator 112 acquires recommendation information records stored in the recommendation information storage 151. Next, the recommended content information creator 112 makes a comparison between the search input item acquired by the search input item acquirer 111 and the search input items of the recommendation information records acquired from the recommendation information storage 151, and then extracts recommendation information records having the matched search input item according to their points. Next, the recommended content information creator 112 creates recommended content information that links to the content indicated by the extracted recommendation information records so that combinations (sets) including the content are displayed to the user in descending order of appearance frequency.

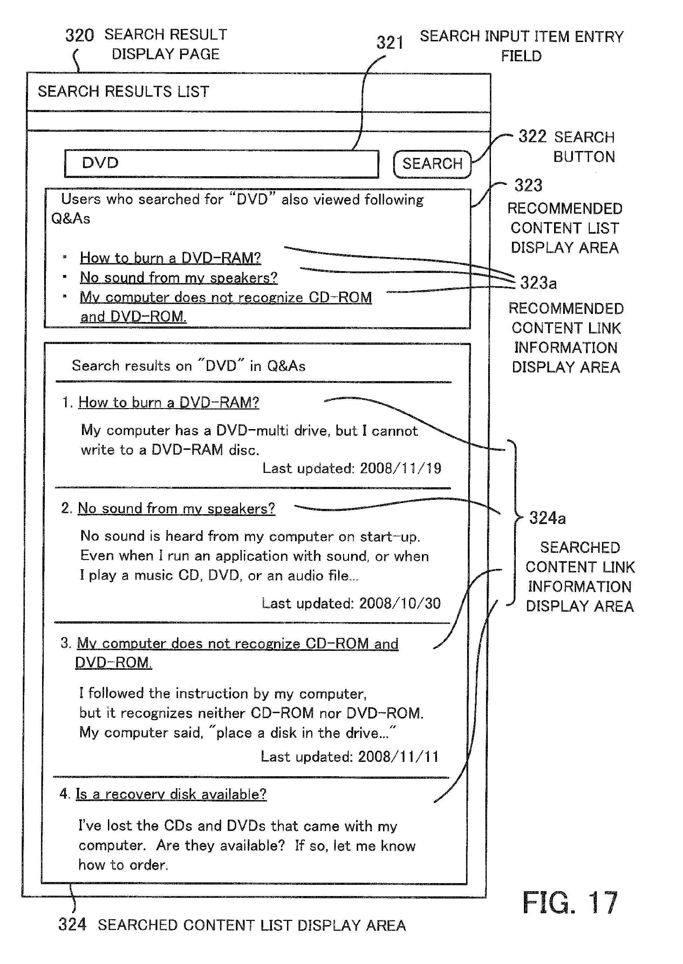

The recommended content information provider 113 provides the user with the recommended content information created by the recommended content information creator 112 together with search result information indicating search results made by the searcher 131. These recommended content information and search result information are provided to the user by being transmitted to the user's information processing apparatus 300 via the Internet 40 as data for displaying a search result display page (to be described later in FIG. 17), which is an web page having URL links. The recommended content information and search result information may be provided in a variety of forms that allow the user to access content, for example, as an e-mail which has links to the content.

The search request receiver 121 receives a search request for providing content search results of a search based on a search input item entered by a user, from the user's information processing apparatus 300 via the Internet 40.

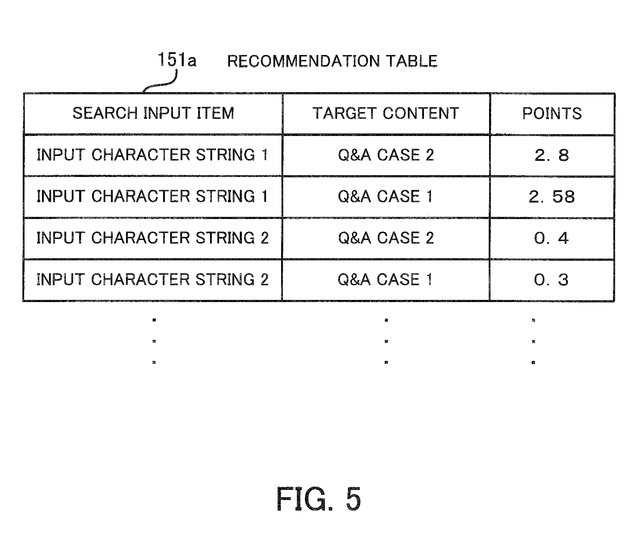

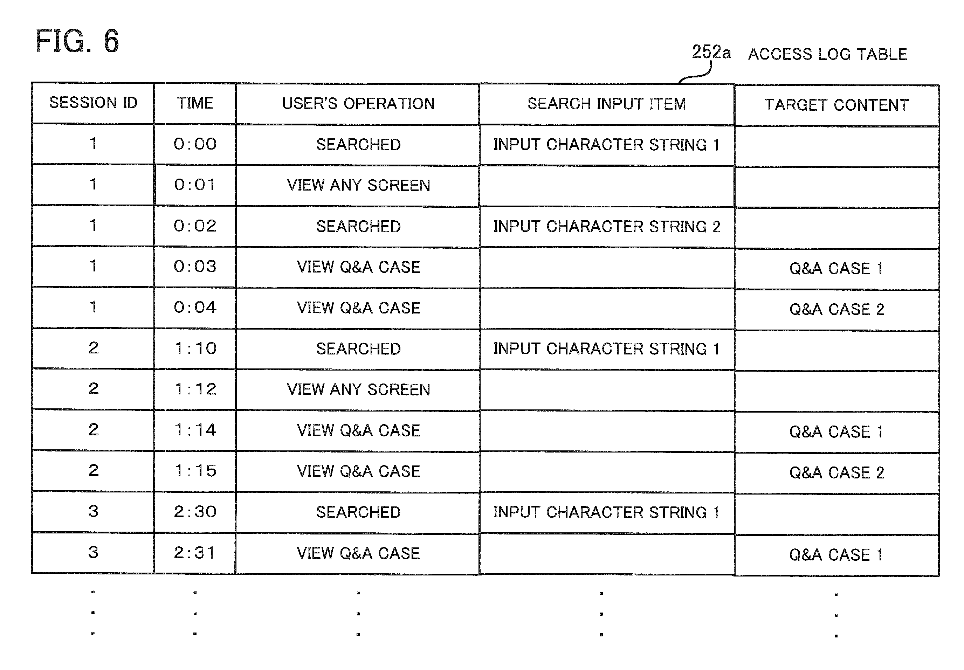

The delivery request receiver 122 receives a delivery request for providing content requested by the user, from the user's information processing apparatus 300 via the Internet 40.
The recommendation information storage 151 stores recommendation information records (to be described later with reference to FIG. 5) each of which associates a combination of content viewed in a session made for content searching and viewing and a search input item used in the session, with points given based on the search input item and the content. The recommendation information records are created based on access log information (to be described later with reference to FIG. 6) stored in an access log information storage 252.

The content information provider 123 provides the user with the requested content data stored in the database server 200, in response to a delivery request received by the delivery request receiver 122 by transmitting to the information processing apparatus 300 via the Internet 40. In this connection, the content data may be provided directly to the user from the database server 200. In addition, the content data may be stored in the web server 100.

The searcher 131 carries out a search for the search input item indicated by the user's search request received by the search request receiver 121. The search result information indicating the search results by the searcher 131, as illustrated in a searched content list display area to be described later with reference to FIG. 17, is provided by the recommended content information provider 113 to the user together with recommended content information.

The database server 200 is a computer that creates a recommendation information record based on a content search request and content delivery request received by the web server 100. The database server 200 includes an access log information creator 241, a recommendation information creator 242, the access log information storage 252, and a content information storage 260. A recommendation information creating method is achieved by causing a computer to function as the database server 200 with a computer program.

The access log information creator 241 acquires search input items from search requests, and also acquires content requested by delivery requests. Next, the access log information creator 241 creates access log information about the acquired search input items and content for each session. Next, the access log information creator 241 stores the created access log information in the access log information storage 252.

The recommendation information creator 242 acquires the access log information from the access log information storage 252. Next, the recommendation information creator 242 gives search points to each search input item in search order within each session indicated in the acquired access log information. Next, the recommendation information creator 242 gives viewing points to each of content in viewing order within the session. The recommendation information creator 242 then gives order points on the basis of the search points given to the search input item of a combination and the viewing points given to the content of the combination.

The recommendation information creator 242 extracts a combination of a search input item searched for first in each session indicated in the acquired access log information and content viewed last in the session. Next, the recommendation information creator 242 gives destination points to the extracted combination according to the appearance frequency thereof.

The recommendation information creator 242 calculates the sum of the order and destination points for each combination. Next, the recommendation information creator 242 creates a recommendation information record that associates the combination of the search input item and content with the summed points, and then stores the created recommendation information record in the recommendation information storage 151.

The recommendation information creator 242 acquires access log information from the access log information storage 252. Next, the recommendation information creator 242 gives search points to each search input item in search order within each session indicated in the acquired access log information. The recommendation information creator 242 then gives viewing points to each of content in viewing order within the session. Next, the recommendation information creator 242 then gives order points on the basis of the search points given to the search input item of a combination and the viewing points given to the content of the combination. The recommendation information creator 242 then creates a recommendation information record indicating points such as the order points given to each combination of a search input item and content. Next, recommendation information creator 242 stores the created recommendation information record in the recommendation information storage 151.

The access log information storage 252 stores access log information which indicates search input items indicated by user's search requests received by the search request receiver 121 and content requested by delivery requests received by the delivery request receiver 122, for each session.

The content information storage 260 stores content data that is to be provided by the content providing system 10 to a user who is looking for useful information. For example, the content providing system 10 provides FAQs as after-sales service for products made by the manufacturer that manages the content providing system 10.

In the content providing system 10 according to the embodiment, the web server 100 has the content recommendation function of recommending content to a user on the basis of recommendation information records, and the database server 200 has the recommendation information creation function of creating the recommendation information records based on acquired access log information. However, a single computer may have both of these content recommendation function and recommendation information creation function. In addition, in the web server 100, any function that a web application causes the content providing system 10 to have according to the embodiment may be achieved by the user's information processing apparatus 300.

In this embodiment, the functions of the content providing system 10 are provided to the user's information processing apparatus 300 via the Internet 40. However, some of the functions may be provided off-line to the user. For example, content having user-needed information may be provided off-line as a help file. In this case, the database server 200 may collect usage logs of the help file to create recommendation information records, and may distribute the recommendation information records to the information processing apparatus 300 so as to allow the user to use the recommendation information records in searching the help file.

Next, a description is given for a table to be used in the embodiment.
FIG. 5 illustrates a configuration of a recommendation table. The recommendation table 151a of FIG. 5 is stored in the recommendation information storage 151, and is created and managed by the web server 100. The recommendation table 151a stores recommendation information records for use in recommending content having needed information to a user on the basis of a search input item entered for content search.

The recommendation table 151a has the following data fields: "Search input item", "Target content", and "Points". In the recommendation table 151a, values in each row are associated with each other as one recommendation information record.

The "Search input item" field contains information entered by a user to search for content having needed information. For example, such information may be a character string such as a keyword related closely to what the user needs.
Specifically, suppose that the web server 100 provides FAQ content as after-sales service for personal computers. If a user has a personal computer with a broken keyboard, the user may enter a character string "keyboard does not work" as a search input item for this problem. The search input item is not limited to a sentence, but may be a word, a product code, a two-dimensional code, another type of information, or a combination thereof. Furthermore, the web server 100 may accept a plurality of search input items at a time.

The "Target content" field indicates content recommended to a user by the web server 100 out of content provided by the web server 100. For example, this "Target content" field contains the name of target content recommended by the web server 100.

The "Point" field contains points given to a combination (hereinafter, referred to as a "set") of a search input item and target content. The points are used by the web server 100 to determine which content to be recommended to a user. The points indicate a possibility that corresponding content may have information needed by a user who enters a corresponding search input item.

In the embodiment, the web server 100 provides a user with both target content (likely to have user-needed information) and nontarget content (for example, "Any screen" to be described later with reference to FIG. 6). However, all content provided by the web server 100 may be taken as target content. Also in the embodiment, the web server 100 recommends target content to a user, and the database server delivers target content to the user and performs analysis on the target content. However, a single server may have these functions. Alternatively, these functions may be assigned to a plurality of different servers in a distributed manner.

FIG. 6 illustrates a configuration of an access log table.
The access log table 252a of FIG. 6 is stored in the access log information storage 252, and is created and managed by the database server 200. Access log information is created and stored when a request made to a web server 100 from a user who uses content provided by the web server 100 is processed. The access log table 252a stores access log information indicating users' operations such as content search to acquire needed information and content accessing based on search results.

The access log table 252a has the following data fields: "Session ID", "Time", "User's operation", "Search input item", and "Target content". In the access log table 252a, values in each row are associated with each other as a piece of access log information.

The "Session ID" field contains a code which is assigned to a user's information processing apparatus 300, 300a, 300b (see FIG. 2) (for example, information processing apparatus 300) when the user accesses the web server 100. The web server 100 identifies a user and session on the basis of such a session ID, analyzes user's operations within the session, and creates recommendation information records (see FIG. 5).

The "Time" field indicates a time of a user's operation. This time is used to detect the order of the user's operations within a session.
The "User's operation" field indicates a type of operation made by a user accessing the web server 100. The type of user's operation is acquired based on a request made to the web server 100 by the user and transmitted from the information processing apparatus 300 having an established session. Example types of user's operation include: "Searched" indicating that a user made a search by entering a character string as a search input item; "View any screen" indicating that a user viewed content other than target content; and "View Q&A case" indicating a user viewed a Q&A tip. "Any screen" refers to a screen other than screens of target content. Specifically, the "any screen" may be a help screen, an advertisement screen, or another kind of screen that provides information other than recommended information.

The "Search input item" field contains a search input item entered by a user for search, which may be a character string. The "Target content" field indicates target content provided by the web server 100 and viewed by a user. The web server 100 analyzes search input items, target content, and information indicated by access log information, and recommends target content to a user on the basis of the analysis result.

Suppose that a user accesses the web server 100 from the information processing apparatus 300. When a session is established, the web server 100 assigns a session ID to the information processing apparatus 300. Every time the web server 100 receives a user's request from the information processing apparatus 300, which has established the session, the web server 100 creates access log information as indicated in each row of the access log table 252a.

Next, processes according to the embodiment will be described.

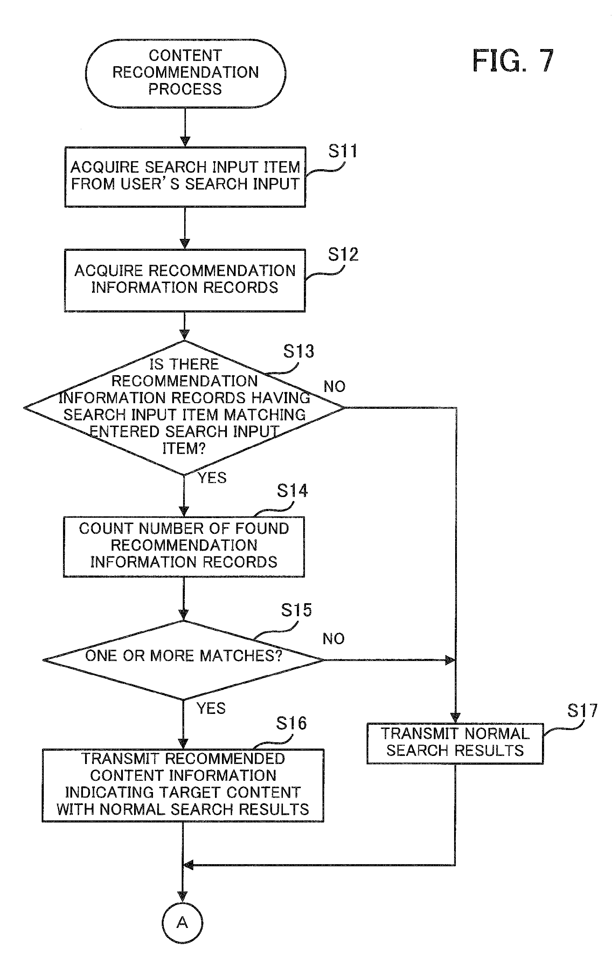

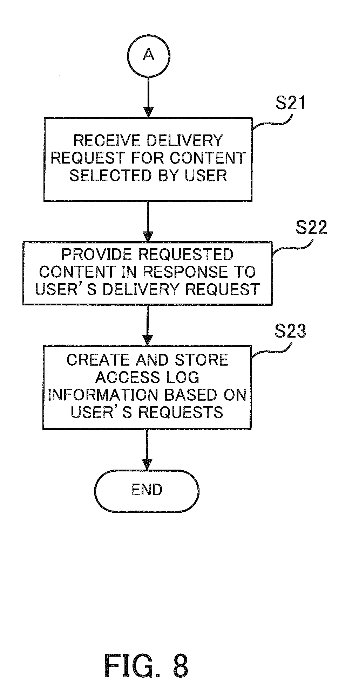

FIGS. 7 and 8 are a flowchart of a content recommendation process. The content recommendation process of FIGS. 7 and 8 is performed by the web server 100 (see FIG. 2) and database server 200 (see FIG. 2) according to the embodiment. The content recommendation process is a process of receiving a search request and a content delivery request made by a user, processing the received request, providing recommended target content, and further generating access log information according to the received request (see FIG. 6). Suppose that a search request is made to search content available on the web server 100 by a user's information processing apparatus 300, 300a, 300b (for example, information processing apparatus 300) having an established session with the web server 100 via the LAN 50 (see FIG. 2), and the search request receiver 121 (see FIG. 4) of the web server 100 receives this request. At this time, the web server 100 starts this content recommendation process. In this case, suppose that a search input item entered by the user is sent together with the search request from the information processing apparatus 300. In addition, the search request transmitted by the user is sent to the access log information creator 241 (see FIG. 4) of the database server 200.

[Step S11] The search input item acquirer 111 (see FIG. 4) acquires a search input item entered by the user, received together with the search request by the search request receiver 121.

[Step S12] The recommended content information creator 112 (see FIG. 4) acquires recommendation information records (see FIG. 5) from the recommendation information storage 151 (see FIG. 4).

[Step S13] The recommended content information creator 112 uses the recommendation information records acquired in step S12 to make a comparison between the search input items of the recommendation information records and the search input item entered by the user and acquired in step S11, and determines whether or not there are recommendation information records having a search input item matching the entered search input item. If there are recommendation information records having the matched search input item, the process advances to step S14. On the other hand, if there is no recommendation information record having the matched search input item, the process advances to step S17.

The matching between search input items may include a partial match, in addition to a perfect match. For example, the partial match detects if the search input item of a recommendation information record is included in a user-entered search input item, or vice versa. Another search criterion may also be employed instead.

[Step S14] The recommended content information creator 112 counts the number of recommendation information records having the search input item matching the user-entered search input item on the basis of the comparison result obtained in step S13.

[Step S15] The recommended content information creator 112 determines whether or not one or more matches have been detected, on the basis of the count result obtained in step S14. When the number of matches is one or greater, the process advances to step S16. On the other hand, when the number of matches is zero, the process advances to step S17.

[Step S16] The recommended content information provider 113 (see FIG. 4) provides the information processing apparatus 300 with the recommended content information regarding the matched search input item obtained by the comparison of step S13, created by the recommended content information creator 112, together with normal content search results obtained based on the user-entered search input item by the searcher 131 (see FIG. 4). In response, the information processing apparatus 300, which is being used by the user, displays a search result display page (to be described later with reference to FIG. 17). After that, the process advances to step S21 (FIG. 8).

[Step S17] The recommended content information provider 113 provides the information processing apparatus 300 with normal content search results made by the searcher 131 based on the user-entered search input item. On the basis of the normal search results, the information processing apparatus 300, which is being used by the user, displays a search result display page (not illustrated). After that, the process advances to step S21.

[Step S21] The delivery request receiver 122 (see FIG. 4) receives a content delivery request transmitted from the information processing apparatus 300 used by the user via the Internet 40 (see FIG. 2). At this time, this delivery request made by the user is transmitted to the access log information creator 241 of the database server 200.

[Step S22] The content information provider 123 (see FIG. 4) provides the user with the requested content. Specifically, the content information provider 123 extracts the content data of the content requested for delivery from the content information storage 260 (see FIG. 4) of the database server 200 connected to the content information provider 123 with the LAN 50, and sends the content data to the user's information processing apparatus 300 via the Internet 40.

[Step S23] The access log information creator 241 stores access log information in the access log information storage 252. The access log information indicates the user's search request received by the search request receiver 121 at the start of the content recommendation process, and the content delivery request received by the delivery request receiver 122 in step S21. After that, the process comes to an end.

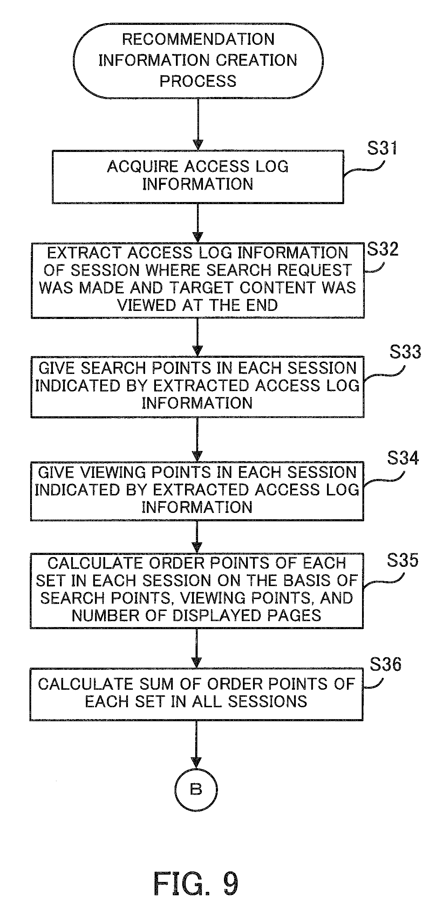

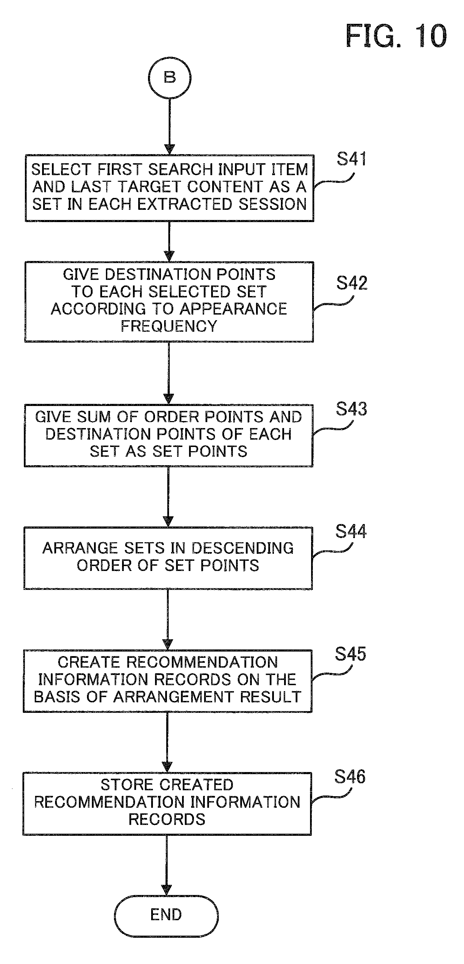

FIGS. 9 and 10 are a flowchart illustrating a recommendation information creation process. The recommendation information creation process of FIGS. 9 and 10 is performed by the database server 200 according to the embodiment. The recommendation information creation process analyzes the access log information (see FIG. 6) created in the content recommendation process described with reference to FIGS. 7 and 8, and creates recommendation information records (see FIG. 5). The recommendation information creation process is started by the database server 200 when a predetermined event occurs, such as a lapse of a certain period of time, or content update.

[Step S31] The recommendation information creator 242 (see FIG. 4) acquires access log information stored in the access log information storage 252 (see FIG. 4).
[Step S32] The recommendation information creator 242 extracts access log information regarding each session in which a search request was made and target content was viewed at the end of the session, out of sessions having access log information.

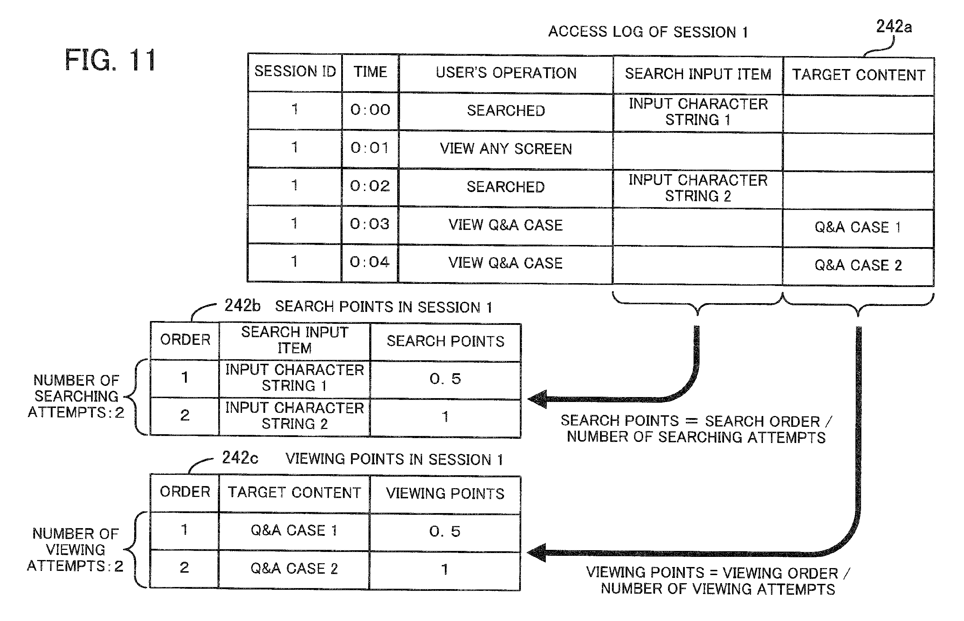

[Step S33] The recommendation information creator 242 gives search points to each search input item entered within each session indicated by the extracted access log information. How to give search points will be described later with reference to FIG. 11.

[Step S34] The recommendation information creator 242 gives viewing points to each of target content viewed within each session indicated by the extracted access log information. How to give viewing points will be described later with reference to FIG. 11.

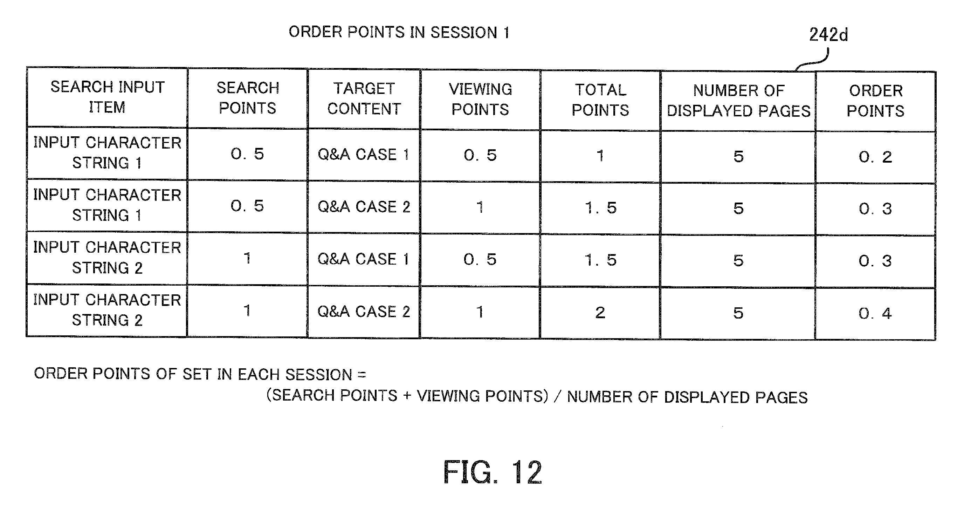

[Step S35] The recommendation information creator 242 calculates the sum of the search and viewing points for each set within each session indicated by the extracted access log information, and divides the sum by the number of displayed pages, thereby calculating order points of the set within the session. How to give order points will be described later with reference to FIG. 12.

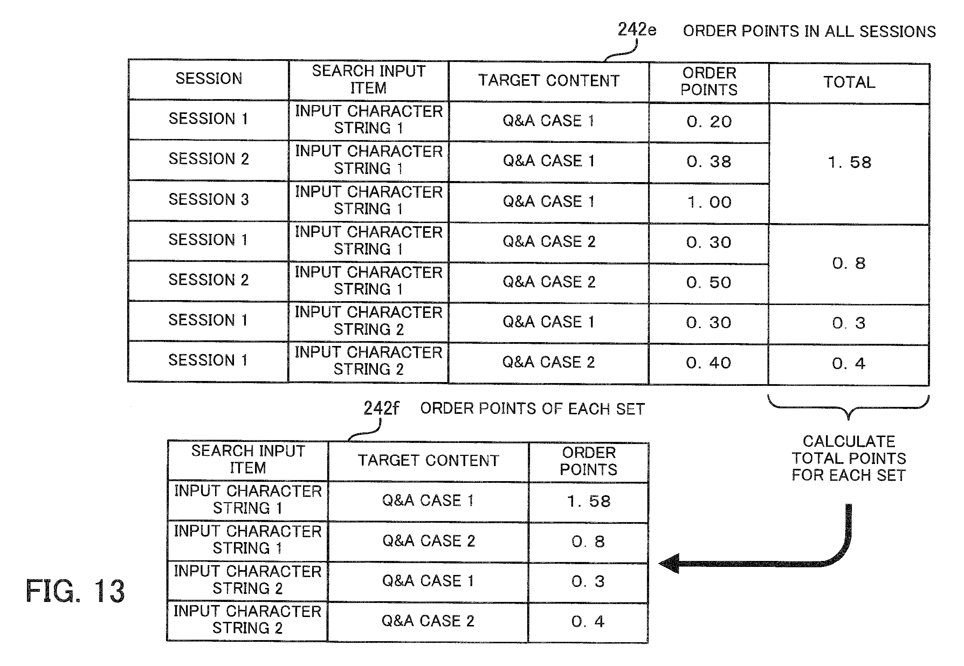

[Step S36] The recommendation information creator 242 calculates the sum of the order points given to the same set in all sessions indicated by the extracted access log information. How to sum up the order points of the set will be described later with reference to FIG. 13. After that, the process advances to step S41 (FIG. 10).

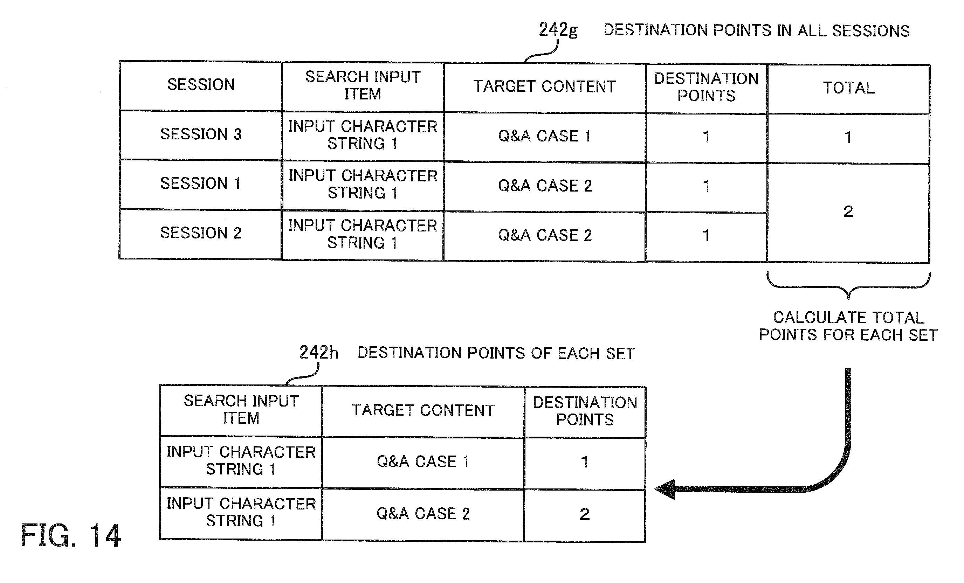

[Step S41] The recommendation information creator 242 selects a search input item entered first by the user and target content viewed last by the user as a set in each session indicated by the access log information extracted in step S32 (FIG. 9). How to determine a set will be described later with reference to FIG. 14.

[Step S42] The recommendation information creator 242 gives destination points to each set selected in step S41 in the sessions, depending on the appearance frequency thereof. How to give destination points will be described later with reference to FIG. 14.

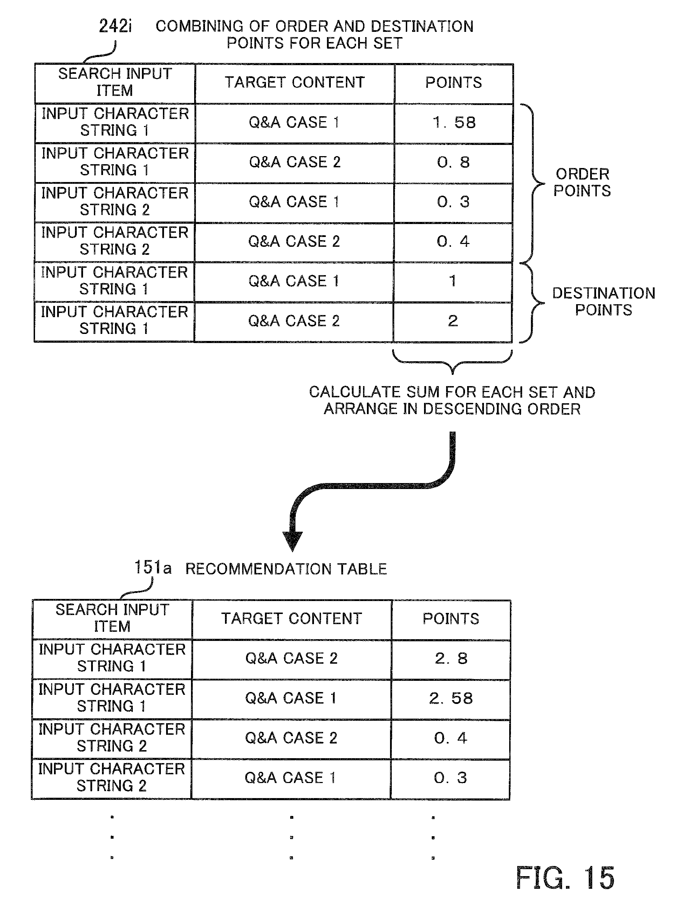

[Step S43] The recommendation information creator 242 calculates the sum of the total order points of each set calculated in step S36 (FIG. 9) and the total destination points of the set calculated in step S42, and sets the calculated result as set points. How to set set points will be described later with reference to FIG. 15.

[Step S44] The recommendation information creator 242 arranges the sets in descending order of set points set in step S43. How to arrange the sets will be described later with reference to FIG. 15.

[Step S45] The recommendation information creator 242 creates recommendation information records (see FIG. 5) based on the arrangement result of the sets in step S44. How to create recommendation information records will be described later with reference to FIG. 15.

[Step S46] The recommendation information creator 242 stores the recommendation information records created in step S45 in the recommendation information storage 151 (see FIG. 4) included in the web server 100. After that, the process comes to an end.

Next, a description is given for how to use databases provided in the content providing system according to the embodiment to create recommendation information records.
FIG. 11 illustrates how to give search points and viewing points within each session. As described with reference to FIGS. 9 and 10, the recommendation information creator 242 (see FIG. 4) acquires access log information (see FIG. 6) to create recommendation information records (see FIG. 5). Next, the recommendation information creator 242 extracts access log information regarding each session in which a search request for searching content provided by the content providing system 10 (see FIG. 4) was made by a user and target content was viewed at the end of the session, like the access log of Session 1 242a illustrated in FIG. 11.

Suppose that there is a session where the user viewed target content, then made another search, and finishes the search. In this case, it is likely that the user did not reach needed information. Therefore, in the embodiment, in order to exclude logs for such sessions, access logs to be extracted are preferably limited to ones regarding each session where any target content was viewed at the end of the session.

Next, referring to FIG. 11, as illustrated in the search points in Session 1 242b, the recommendation information creator 242 sets search points based on the access log information of Session 1. Specifically, the recommendation information creator 242 extracts search input items recorded in the log information on search requests (a row where "Searched" is set as "User's operation"), from the extracted access log 242a. The extracted search input items are arranged based on "Time" in the order of search-requesting. The recommendation information creator 242 sets search points obtained by the following formula to each of the search input item arranged in the order: $S ⁢ e ⁢ a ⁢ r ⁢ c ⁢ h p ⁢ o ⁢ int ⁢ s = S ⁢ e ⁢ a ⁢ r ⁢ c ⁢ h o ⁢ r ⁢ d ⁢ e ⁢ r / N ⁢ u ⁢ m ⁢ b ⁢ e ⁢ r o ⁢ f s ⁢ e ⁢ a ⁢ r ⁢ c ⁢ h ⁢ i ⁢ n ⁢ g a ⁢ t ⁢ t ⁢ e ⁢ m ⁢ p ⁢ t ⁢ s$

According to the log regarding search requests in Session 1, "Input character string 1" in Session 1 was entered at the earliest time, so the search order thereof is "1". In addition, two search requests were made in Session 1. Therefore, the number of searching attempts is "2". Thus, search points given to "Input character string 1" are 1/2 = 0.5. In this connection, the later a search input item is entered, the larger search points are given. This is based on the idea that a search input item entered later is more important because the item is likely to be closer to user-needed information.

Similarly, referring to FIG. 11, as illustrated in the Viewing points in Session 1 242c, the recommendation information creator 242 sets viewing points based on the access log information of Session 1. Specifically, the recommendation information creator 242 identifies the target content recorded on the log information on viewing of target content (for example, Q&A case) (in the row where "View Q&A case" is set in "User's operation"), from the extracted access log 242a. The recommendation creator 242 then arranges the identified target content based on the "time" in the order of search-requesting. The recommendation information creator 242 then sets viewing points obtained by the following formula to each of the target content arranged in the order: $V ⁢ i ⁢ e ⁢ w ⁢ i ⁢ n ⁢ g p ⁢ o ⁢ int ⁢ s = V ⁢ i ⁢ e ⁢ w ⁢ i ⁢ n ⁢ g o ⁢ r ⁢ d ⁢ e ⁢ r / N ⁢ u ⁢ m ⁢ b ⁢ e ⁢ r o ⁢ f V ⁢ i ⁢ e ⁢ w ⁢ i ⁢ n ⁢ g a ⁢ t ⁢ t ⁢ e ⁢ m ⁢ p ⁢ t ⁢ s$

According to the log information on delivery requests in Session 1, "Q&A case 2" in Session 1 was requested at the second earliest time. Therefore, the order thereof is "2". Since two delivery requests were made in Session 1, the number of viewing attempts is "2". Therefore, viewing points given to the "Q&A case 2" are 2/2 = 1. In other words, similarly to search points, the later target content is requested, the larger viewing points are given. When the user views content having user-needed information, the user will finish the viewing attempt and will not view content any further. Therefore, it is thought that content viewed earlier would not have user-needed information. This is based on the idea that target content viewed later is more important because such target content is likely to have user-needed information.

FIG. 12 illustrates how to give order points in each session. Referring to FIG. 12, as illustrated in the Order points in Session 1 242d, the recommendation information creator 242 (see FIG. 4) sets order points based on the access log information of Session 1. Specifically, as described earlier with reference to FIG. 11, the recommendation information creator 242 sets order points obtained by the following formula to each combination (set) of a search input item and target content of Session 1 on the basis of the access log information of Session 1, by using the search points given to the search input item and the viewing points given to the target content: $O ⁢ r ⁢ d ⁢ e ⁢ r p ⁢ o ⁢ int ⁢ s o ⁢ f s ⁢ e ⁢ t i ⁢ n e ⁢ a ⁢ c ⁢ h s ⁢ e ⁢ s ⁢ s ⁢ i ⁢ o ⁢ n = \left(S⁢e⁢a⁢r⁢c⁢h p⁢o⁢int⁢s + V⁢i⁢e⁢w⁢i⁢n⁢g p⁢o⁢int⁢s\right) / N ⁢ u ⁢ m ⁢ b ⁢ e ⁢ r o ⁢ f d ⁢ i ⁢ s ⁢ p ⁢ l ⁢ a ⁢ y ⁢ e ⁢ d p ⁢ a ⁢ g ⁢ e ⁢ s$ Specifically, the recommendation information creator 242 sets a value obtained by dividing the sum of the search points given to the search input item of a set and the viewing points given to the target content value of the set, by the "number of displayed pages" indicating how many pages were displayed in Session 1, as order points of the set. In this connection, the number of displayed pages may be the sum of the number of search requests and the number of delivery requests for target content and other content, indicated in the access log information of a session in question (for example, Session 1).

Regarding a set of "input character string 1" and "Q&A case 1" in Session 1, for example, the search points given to the "input character string 1" in Session 1 are "0.5". The viewing points given to the "Q&A case 1" in Session 1 are "0.5". Therefore, the sum of these points is "1". Then, a value "0.2" obtained by dividing the sum by the number of displayed pages "5" is set as order points given to the set of the "input character string 1" and the "Q&A case 1".

FIG. 13 illustrates order points of each set. As illustrated in the Order points in all sessions 242e of FIG. 13, the recommendation information creator 242 (see FIG. 4) calculates the sum of the order points of each set in all sessions. The order points in all sessions are obtained in the same way as in Session 1 of FIG. 12. The calculated results are illustrated in the order points of each set 242f in FIG. 13.

FIG. 14 illustrates destination points of each set. As illustrated in the destination points in all sessions 242g of FIG. 14, the recommendation information creator 242 (see FIG. 4) calculates the sum of the destination points given to a set in all sessions. Destination points are points given to a set of a search input item indicated by the first search request and target content requested by the last delivery request in each session. This is based on the idea that the last target content viewed by a user is likely to have user-needed information and that a set of the user-needed information and a search input item entered first should be focused on so that other users can reach the needed information in an earlier stage. The destination points calculated for each set are illustrated in the destination points of each set 242h in FIG. 14.

As described earlier with reference to FIG. 11, in the embodiment, access log information regarding only sessions where target content was viewed at the end of a session is extracted.

FIG. 15 illustrates how to create recommendation information records. As illustrated in the combination of order and destination points for each set 242i of FIG. 15, the recommendation information creator 242 (see FIG. 4) combines the order points (see FIGS. 11 to 13) and the destination points (see FIG. 14) of each set. Next, the recommendation information creator 242 calculates the sum of these points for each set based on the combined result, and then arranges the sets in descending order of the calculated points. As described with reference to FIG. 5, the recommendation information creator 242 then stores thus obtained sets and their points in the recommendation information storage 151 (see FIG. 4) as recommendation information records, as illustrated in a recommendation table 151a of FIG. 15.

Next, a description is given for a display screen to be displayed in the embodiment.

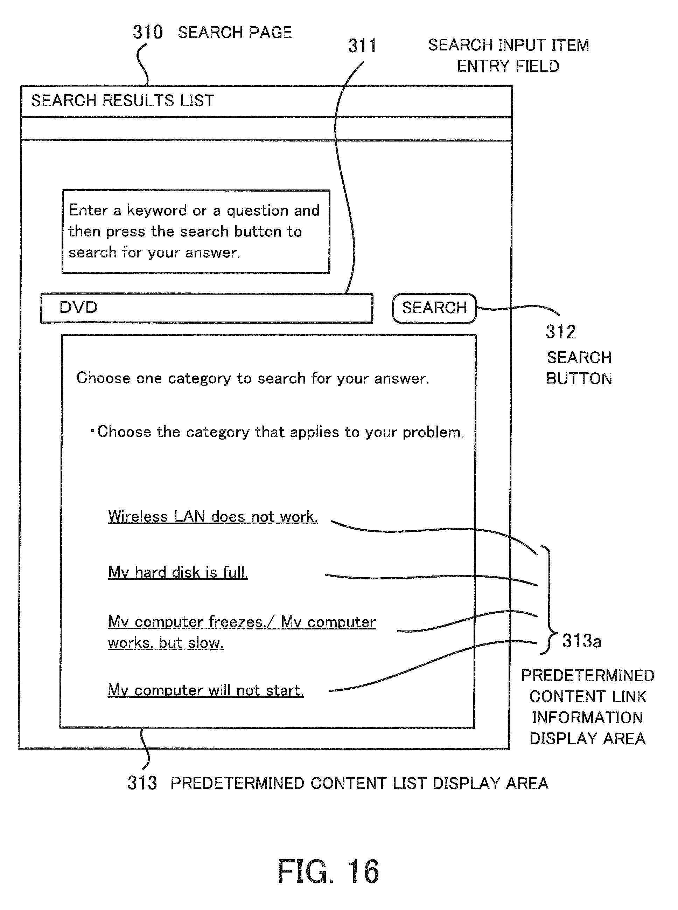

FIG. 16 illustrates a search page. A search page 310 of FIG. 16 is a web page that is displayed by a browser on a monitor (not illustrated) connected to an information processing apparatus 300 (see FIG. 2) used by a user. The search page 310 is an example search page where a user enters a search input item to search target content provided by the content providing system 10 (see FIG. 4) so as to access needed information.

The search page 310 is a page where a user who wants to search target content provided by the content providing system 10 to obtain needed information enters a search input item such as a search character string for content search. The search page 310 includes a search input item entry field 311, a search button 312, and a predetermined content list display area 313. The predetermined content list display area 313 has a predetermined content link information display area 313a.

The search input item entry field 311 is an entry field into which a user who wants to search target content with a search input character string enters a search input item. The search button 312 is a button to be pressed by the user, who wants to search target content with a search input character string, after the user enters a search input item. When the user enters the search input item in the search input item entry field 311, and then presses the search button 312, the searcher 131 (see FIG. 4) searches target content in order for the user to access user-needed information. In addition, on the basis of the entered search input item, through the content recommendation process illustrated in FIGS. 7 and 8, the content providing system 10 recommends content to the user, as illustrated in a search result display page to be described later with reference to FIG. 17.

In the predetermined content list display area 313, the predetermined content link information display area 313a has links to content which the manager of the content providing system 10 takes to be important and frequently used, or content taken to be important based on the number of user accesses. The predetermined content list display area 313 enables the user to access the needed information in an intuitive way without making a search. The content to be linked on the predetermined content link information display area 313a may have user-needed information, or may be provided in a directory structure so that the user makes a selection in plural steps to access the needed information.

FIG. 17 illustrates a search result display page. A search result display page 320 of FIG. 17 is a web page that is displayed by a browser on a monitor (not shown) connected to an information processing apparatus 300 (see FIG. 2) used by a user. The search result display page 320 is an example search result display page which displays search results that are obtained by the searcher 131 (see FIG. 4) which searches target content provided by the content providing system 10 (see FIG. 4) with a search input item entered by a user on the search page 310 (see FIG. 16).

The search result display page 320 is a page which displays results of searching target content with a search input item such as a search character string entered by a user. The search result display page 320 includes a search input item entry field 321, a search button 322, a recommended content list display area 323, and a searched content list display area 324. The recommended content list display area 323 has a recommended content link information display area 323a. The searched content list display area 324 has a searched content link information display area 324a.

The search input item entry field 321, similarly to the search input item entry field 311 (see FIG. 16), is an entry field into which a user who wants to search target content with a search input character string enters a search input item. The search input item entry field 321 displays the search input item entered by the user in the search input item entry field 311 so that the user can make sure that the search input item is proper. The search button 322, similarly to the search button 312 (see FIG. 16), is a button to be pressed by the user after the user who wants to search target content with a search input character string enters a search input item. When the user is not satisfied with the search results displayed on the search result display page 320, the user is able to make another search with a different search input item by using the search input item entry field 321 and the search button 322. In this case, based on this entered search input item, the content recommendation process of FIGS. 7 and 8 is repeated so that the content providing system 10 recommends other content to the user once again.

The recommended content list display area 323 displays links to content recommended to the user by the content recommendation function of the content providing system 10, in the recommended content link information display area 323a, in descending order of points. In this way, on the basis of the search input item entered by the user to search for needed information, the recommended content list display area 323 displays recommend content which is likely to have needed information, separately from the normal search results.

As illustrated in FIG. 17, the recommended content list display area 323 displays, for example, a message "Users who searched for "DVD" also viewed following Q&As" and the links to several recommended Q&A content. The user can access desired content by clicking the link thereof.

The recommended content list display area 323 is preferably displayed under the search input item entry field 321 and above the searched content list display area 324 so that the recommended content list display area 323 is easily viewable and jumps to the eyes of the user. When the user finds any content that is considered to include needed information in the recommended content list display area 323, the user clicks the link thereto to access the content quickly.

The searched content list display area 324 displays links to the content found by the searcher 131 of the content providing system 10 in the searched content link information display area 324a. Therefore, the content found by searching for the needed information can be provided to the user.

The content recommendation function according to the embodiment is not incompatible with a normal search function; it is a system which enables a user to access content having needed information with greater accuracy, based on what content other users searched for and viewed. Therefore, as illustrated in FIG. 17, both recommended content provided by the content recommendation function according to the embodiment and content found by the normal search function may be displayed at the same time to the user. Alternatively, only recommended content may be displayed. In the embodiment, recommended content link information is displayed on a web page. However, such recommended content link information may be provided by another communication method such as via e-mail.

As has been described above, in the embodiment, search input items entered by other users for content search and target content viewed last by these users are extracted and used for recommending content to a user. As a result, it is possible to provide, on the basis of a search input item entered by the user, the user with content frequently viewed by other users who entered the same search input item. This enables content having user-needed information to be recommended to a user with great accuracy.

Suppose that a user quits searching in the middle of the search but finally reached content having needed information by another means (for example, the content recommendation function according to the embodiment or the predetermined content link information display area 313a in FIG. 16). Even in this case, the user's actions are reflected on recommendation information records. The user's action taken after the search is also taken into consideration. Therefore, it is possible to recommend content based on search input items entered first for search and content viewed last, with taking users' actions after search into consideration.

A search criteria different from the one in normal search is employed in the embodiment for content recommendation. Therefore, even when a user has difficulty in finding needed information through search, it is possible that content having needed information is recommended to the user.

The above processing functions are achieved by a computer. In this case, a program which describes the processing functions of the web server 100 and the database server 200 is provided. When the program is executed by a computer, processing functions are achieved on the computer.

The description program may be stored in a computer-readable recording medium. Examples of computer-readable recording media include magnetic recording devices, optical discs, magneto-optical recording media, and semiconductor memories. Examples of magnetic recording devices include HDDs, flexible disks (FD), and magnetic tapes (MT). Examples of optical discs include DVDs (digital versatile disc), DVD-RAMs, CD-ROMs (compact disc-read only memory), CD-R (recordable)/RW(ReWritable). Examples of magneto-optical recording media include magneto-optical disks (MO).

For distribution of the program, the program is sold in a portable form such as a DVD or a CD-ROM. Alternatively, the program is stored in a server computer, and then transferred from the server computer to another computer through a network.

In order to run the program, for example, a computer stores, in the local storage, the program recorded in a portable recording medium or the program transferred from the server computer. The computer then reads the program from the storage to perform processes according to the program. The computer may read a program directly from a portable recording medium to perform the processes according to the program. Alternatively, the computer may perform one process after another according to the program every time a program is transferred from the server computer.

The disclosed content recommending method, recommendation information creating method, content recommendation program, content recommendation server, and content providing system have been described according to the illustrated embodiment. However, each element may be replaced by any other element having equivalent functions. Any other configuration or step may be added thereto. In addition, two or more configurations in the embodiment may be combined to provide the disclosed techniques.

The foregoing is considered as illustrative only of the principles of the present invention. Further, since numerous modifications and changes will readily occur to those skilled in the art, it is not desired to limit the invention to the exact construction and applications shown and described, and accordingly, all suitable modifications and equivalents may be regarded as falling within the scope of the invention in the appended claims and their equivalents.

### Description of Reference Numerals

1 content recommendation server
1a search input item acquisition means
1b recommended content information creation means
1c recommended content information providing means
1d recommendation information storage means

## Claims

1. A content recommending method to be executed by a computer, the method comprising:
a search input item acquisition step of acquiring a search input item entered by a user to search content available to the user;
a recommended content information creation step of acquiring, from recommendation information storage means, recommendation information records each associating a combination of content and a search input item with a point given based on the search input item searched for and content viewed in a session where the content was searched for and viewed, making a comparison between the acquired search input item and search input items of the recommendation information records acquired from the recommendation information storage means, to extract recommendation information records having a matched search input item according to points thereof, and creating recommended content information that links to content indicated by the extracted recommendation information records; and
a recommended content information providing step of providing the created recommended content information to the user.

2. The content recommending method according to claim 1, further comprising:
a search request receiving step of receiving a search request for search results of searching the available content with the search input item entered by the user;
a delivery request receiving step of receiving a delivery request for delivering the content to the user; and
an access log information creation step of acquiring the search input item entered for search in the search request and the content requested to be delivered in the delivery request, creating access log information indicating the acquired search input item and content, for each session, and storing the created access log information in access log information storage means, wherein:
the recommendation information records are created based on the access log information stored in the access log information storage means, and
in the search input item acquisition step, the search input item is acquired from the search request received in the search request receiving step.

3. The content recommending method according to claim 2, further comprising a recommendation information creation step of acquiring the access log information from the access log information storage means, extracting a combination of a search input item searched for first and content viewed last in a session indicated in the acquired access log information, giving the point to the extracted combination according to an appearance frequency of the combination, creating a recommendation information record associating the search input item and the content of the combination with the point given to the combination, and storing the created recommendation information record in the recommendation information storage means.

4. The content recommending method according to claim 2, further comprising a recommendation information creation step of acquiring the access log information from the access log information storage means, giving a search point to each search input item in search order within a session indicated in the acquired access log information, giving a viewing point to each of content in viewing order in the session, giving the point to the combination based on the search point given to the search input item of the combination and the viewing point given to the content of the combination, creating a recommendation information record associating the search input item and the content of the combination with the point given to the combination, and storing the created recommendation information record in the recommendation information storage means.

5. The content recommending method according to claim 1, wherein, in the recommended content information creation step, the recommendation information records stored in the recommendation information storage means are acquired, a comparison is made between the search input item acquired in the search input item acquisition step and the search input items of the recommendation information records acquired from the recommendation information storage means to extract recommendation information records having a matched search input item value according to an appearance frequency of the combination based on the point of the combination, and recommended content information that links to the content indicated by the extracted recommendation information records is created so that the content is displayed to the user in descending order according to the appearance frequency of the combination.

6. A recommendation information creating method to be executed by a computer, the method comprising:
an access log information creation step of acquiring a search input item entered by a user to search content available to the user in a search request made for requesting search results, and content requested to be delivered in a delivery request made for requesting delivery of the content, creating access log information recording the acquired search input item and the content for each session where content was searched for and viewed, and storing the created access log information in access log information storage means; and
a recommendation information creation step of acquiring the access log information from the access log information storage means, extracting a combination of a search input item searched for first and content viewed last in a session indicated in the acquired access log information, giving a point of a recommendation information record to the extracted combination according to an appearance frequency of the combination, creating a recommendation information record indicating the search input item and the content of the combination and the point given to the combination, and storing the created recommendation information record in recommendation information storage means.

7. A content recommendation program causing a computer to perform as:
search input item acquisition means that acquires a search input item entered by a user to search content available to the user;
recommended content information creation means that acquires, from recommendation storage means, recommendation information records each associating a combination of content and a search input item with a point given based on the search input item searched for and content viewed in a session where the content was searched for and viewed, making a comparison between the acquired search input item and search input items of the recommendation information records acquired from the recommendation information storage means, to extract recommendation information records having a matched search input item according to points thereof, and creating recommended content information that links to content indicated by the extracted recommendation information records; and
recommended content information providing means that provides the created recommended content information to the user.

8. A content recommendation server comprising:
recommendation information storage means which stores recommendation information records each associating a combination of content to be provided to a user and a search input item entered by the user to search the content with a point given based on the search input item searched for and the content viewed in a session where the content was searched for and viewed;
search input item acquisition means which acquires the search input item entered by the user;
recommended content information creation means which acquires the recommendation information records from the recommendation information storage means, makes a comparison between the search input item acquired by the search input item acquisition means and search input items of the recommendation information records acquired from the recommendation information storage means, to extract recommendation information records having a matched search input item according to points thereof, and creates recommended content information that links to content indicated by the extracted recommendation information records; and
recommended content information providing means which provides the recommended content information created by the recommended content information creation means to the user.

9. A content providing system comprising:
recommendation information storage means which stores recommendation information records each associating a combination of content to be provided to a user and a search input item entered by the user to search the content with a point given based on the search input item searched for and the content viewed in a session where the content was searched for and viewed;
search input item acquisition means which acquires the search input item entered by the user;
recommended content information creation means which acquires the recommendation information from the recommendation information storage means, makes a comparison between the search input item acquired by the search input item acquisition means and search input items of the recommendation information records acquired from the recommendation information storage means, to extract recommendation information records having a matched search input item according to points thereof, and creates recommended content information that links to content indicated by the extracted recommendation information records;
recommended content information providing means which provides the recommended content information created by the recommended content information creation means to the user;
an access log information storage means which stores access log information for each session, indicating the search input item entered for search in a search request received by the search request receiving means and content requested to be delivered in a delivery request received by delivery request receiving means;
recommendation information storage means which stores recommendation information records each associating a combination of the content and the search input item with the point given based on the search input item searched for and the content viewed in a session where the content was searched for and viewed;
access log information creation means which acquires the search input item requested for search in the search request received by the search request receiving means and the content requested to be delivered in the delivery request received by the delivery request accepting means, creates the access log information indicating the acquired search input item and the content for each session, and stores the created access log information in the access log information storage means; and
recommendation information creation means which acquires the access log information from the access log information storage means, extracts a combination of a search input item searched for first and content viewed last in a session indicated in the acquired access log information, gives a point to the extracted combination according to an appearance frequency of the combination, creates a recommendation information record indicating the search input item and the content of the combination and the point given to the combination, and stores the created recommendation information record in the recommendation information storage means.
